# EUROPÄISCHE PATENTSCHRIFT

(11) **EP 1 937 195 B1**
(45) Veröffentlichungstag und Bekanntmachung des Hinweises auf die Patenterteilung: **20.03.2013**
(21) Anmeldenummer: 06805399.0
(22) Anmeldetag: 10.10.2006
(51) Int. Cl.: A61F 5/56

(54) **INTRAORALE VORRICHTUNG**
INTRAORAL DEVICE
DISPOSITIF INTRABUCCAL

(30) Priorität: 13.10.2005 DE 102005049409
(43) Veröffentlichungstag der Anmeldung: 02.07.2008
(73) Patentinhaber: Hoffknecht, Rüdiger, 97084 Würzburg (DE)
(72) Erfinder: Hoffknecht, Rüdiger, 97084 Würzburg (DE)
(74) Vertreter: von den Steinen, Axel
(86) Internationale Anmeldenummer: PCT/DE2006/001781
(87) Internationale Veröffentlichungsnummer: WO 2007/042005

(56) Entgegenhaltungen:
- WO-A-98/41175
- US-A- 4 505 672
- US-A- 5 499 633
- US-A- 5 642 737
- US-B1- 6 170 485

## Beschreibung

Die Erfindung betrifft eine intraorale Vorrichtung, insbesondere zum Einsatz bei schlafbedingten Atmungsstörungen mit einer im Wesentlichen U-förmig ausgebildeten Oberkieferschiene, die auf den oberen Zahnbogen eines Benutzers diesen zumindest bereichsweise umgebend aufsetzbar ist, und einer ebenfalls im Wesentlichen U-förmig ausgebildeten Unterkieferschiene, die auf den unteren Zahnbogen eines Benutzers diesen zumindest bereichsweise umgebend aufsetzbar ist, wobei die Ober- und die Unterkieferschiene jeweils zueinanderweisende Bissflächenbereiche aufweisen, in deren Bereich zumindest eine Fixiereinrichtung vorgesehen ist, mit der die Unterkieferschiene in einer vorbestimmten relativen Position zur Oberkieferschiene fixierbar ist.

Vorrichtungen der eingangs genannten Art, betreffen das Gebiet der Mundorthesen, die insbesondere zur Behandlung des Schnarchens und/oder der Schlafapnoe vorgesehen sind. Bei diesen Erkrankungen liegt eine teilweise oder vollständige Blockade der Atemwege vor, meist in Folge der Entspannung des Körpers im Schlaf. Bei den meisten Betroffenen ist das Auftreten abhängig von der Körperlage. So kommt es häufig in der Rückenlage, wenn der Unterkiefer des Schlafenden nach dorsal gleitet, zum Schnarchen oder zur Apnoe. Registrieren die entsprechenden körpereigenen Sensoren einen durch die erschwerte Atmung hervorgerufenen Sauerstoffmangel, kommt es zu einer Weckreaktion, häufig verbunden mit einer Positionsänderung. Häufig haben Schnarcher und Apnoeiker keinen erholsamen Schlaf, da die Weckreaktionen vor allem die wichtigen Tiefschlafphasen verhindern. Abgeschlagenheit, Nervosität und Müdigkeit auch nach langem Schlaf sind im Wachzustand die Folge.

Die US 5,642,737 offenbart zur Lösung des oben beschriebenen Problems eine Vorrichtung mit einer U-förmig ausgebildeten Oberkieferschiene und einer U-förmig ausgebildeten Unterkieferschiene, wobei auf den zueinanderweisenden Bissflächenbereichen der Ober- und Unterkieferschiene eine Fixiereinrichtung in Form eines Klett- beziehungsweise Haftverschlusses vorgesehen ist. Diese Vorrichtung verfolgt das Ziel, die Ober- und Unterkieferschiene gegeneinander in einer relativen Position zu fixieren und so ein Zurückrutschen beziehungsweise Verschieben des Unterkiefers zu verhindern.

Nachteilig an der aus dem Stand bekannten Vorrichtung ist jedoch, dass die offenbarte Fixiereinrichtung nur eine unzureichende Fixierung der Ober- und Unterkieferschiene zueinander gewährleistet, da bei der die Vorrichtung benutzenden Person während der Schlafphase unbewusst erhebliche Kräfte auf die Ober- beziehungsweise Unterkieferschiene einwirken können und somit keine ausreichende Fixierung gewährleistet werden kann.

Die US 5,49,633 offenbart eine intraorale Vorrichtung gemäß den Merkmalen des Oberbegriffs des Anspruchs 1 zur Fixierung des Unterkiefers, um Schnarchen zu vermeiden. Die Vorrichtung umfasst eine Oberkieferschiene und eine Unterkieferschiene, die jeweils U-förmig ausgebildet sind und auf den zahnseitigen Oberflächen einen furchenartigen Aufnahmebereich für die Zähne aufweisen. Eine der Schienen trägt die maskulinen und die jeweils andere Schiene die femalen Verbindungselemente zur Herstellung einer Steckverbindung zwischen der Ober- und der Unterkieferschiene. Zur individuellen Anpassung der Vorrichtung an einen Patienten sind die Aufnahmebereiche mit einer aus formbare Material bestehenden Schicht versehen, mit der ein Abdruck des Zahnbildes herstellbar ist.

Nachteilig sind bei den Ausführungen aus dem Stand der Technik, dass jeweils zur Anpassung an das individuelle Gebiss zusätzlich zur eigentlichen Schiene ein formbares Material eingebracht wird. Wenngleich mittels dieser Lösung eine langlebige stabile Ausführung erreicht werden kann, sind die Herstellungs- und individuellen Anpassungskosten für derartige Zahnschienen vergleichsweise hoch. Insbesondere für den kurzzeitigen oder testweisen Einsatz verschließen sich daher entsprechende Ausführungsformen der Anwendung.

Der vorliegenden Erfindung liegt daher die Aufgabe zugrunde, eine Vorrichtung zu schaffen, die zum einen in besonderem Maße einfach und kostengünstig herzustellen ist und darüber hinaus eine sichere Fixierung der Ober- und Unterkieferschiene zueinander gewährleistet.

Diese Aufgabe wird durch eine Vorrichtung mit den Merkmalen des Anspruchs 1 gelöst.

Bei einer gattungsgemäßen Vorrichtung weist die Fixiereinrichtung zumindest ein Federelement und zumindest ein das Fixierelement zumindest bereichsweise aufnehmendes Aufnahmeelement auf, wobei das Fixierelement an der Oberkieferschiene und das Aufnahmeelement an der Unterkieferschiene oder, umgekehrt, das Fixierelement an der Unterkieferschiene und das Aufnahmeelement an der Oberkieferschiene vorgesehen ist, wobei Fixierelement und Aufnahmeelement eine im Wesentlichen formschlüssige Steckverbindung bilden. Dies hat den Vorteil, dass eine Fixiereinrichtung geschaffen wird, die einen außerordentlich sicheren Sitz der Ober- und Unterkieferschiene zueinander gewährleistet. Die mit Hilfe der Fixiereinrichtung vorbestimmte Position hält auch stärkeren Verschiebungen der Kieferhälften, beispielsweise während der Schlafphase, sicher Stand und gewährleistet somit die Fixierung der vorbestimmten Position.

Erfindungsgemäß wird nurmehr die Ober- und/oder Unterkieferschiene einschließlich der Fixier- und/oder Aufnahmeelemente jeweils einstückig hergestellt.

Das Aufnahmeelement und/oder das Fixierelement selber muss hierbei in seiner Schiene, vorzugsweise im Bereich der Bissfläche, unmittelbar eingeformt sein. Das Aufnahmeelement und/oder das Fixierelement ist somit ein Bestandteil der Schiene selber.

Zur Gewährleistung der Funktion beim Tragen der Zahnschienen sowie zur Ermöglichung der individuellen Anpassung ist es in der erfinderischen Ausführung erforderlich, dass die die Zahnbögen aufnehmenden Bereiche der Ober- und/oder Unterkieferschiene aus einer verformbaren Formmasse ausgebildet sind. Erforderlich ist hierbei bei Raumtemperatur ein fester, mäßig harter Materialzustand, wobei hingegen bei Erwärmung die eine niedrige Erweichungstemperatur aufweisende thermoplastische Formmasse plastifizierbar sein muss. Das Anpassen beziehungsweise Anlegung der Schienen erfolgt dann durch einfaches Anpressen der erweichbaren Aufnahmebereiche an den Zahnbogen der zu behandelnden Person, um eine Form beziehungsweise einen Abdruck zu erreichen, der exakt dem Zahnbogen des Patienten entspricht.

Die erfindungsgemäße Ausbildung ermöglicht eine besonders einfache und kostengünstige Herstellung der Vorrichtung. So kann die Vorrichtung zum einen zur Unterstützung der Diagnostik und Therapieplanung und zum anderen als günstiges Testgerät zur Feststellung der Akzeptanz und Verträglichkeit eines intraoralen Therapiegeräts verwendet werden.

Das Fixierelement kann im Wesentlichen pilz- und/oder zapfenartig ausgebildet sein und nach Art einer lösbaren Rastverbindung in das zugeordnete Aufnahmeelement eingesteckt werden. Die pilz- und/oder zapfenartige Ausbildung des Fixierelements hat den Vorteil, dass ein besonders sicherer Sitz des Fixierelements im Aufnahmeelement gewährleistet werden kann.

Die für die Funktion der Vorrichtung notwendige Verschlüsselung der beiden Schienen zueinander in der diagnostisch-/therapeutisch gewünschten Position kann auch in der Art ausgebildet sein, als dass es denkbar ist, die Steckverbindung im Wesentlichen als druckknopfartige Verbindung auszubilden. Auch hier kann ein besonders sicherer Sitz der Oberkieferschiene zur Unterkieferschiene gewährleistet werden.

Eine weitere Ausgestaltung der Erfindung sieht vor, dass mindestens zwei, insbesondere zwei bis zehn Fixierelemente auf der Ober- und/oder Unterkieferschiene vorgesehen sein können. Es ist somit möglich die auftretenden Kieferkräfte bei Verschiebung der beiden Schienen zueinander gleichmäßig auf der Ober- und/oder Unterkieferschiene zu verteilen und somit einen außerordentlich positiven Effekt zur Verschlüsselung beziehungsweise Fixierung der beiden Schienen zueinander zu erreichen.

Dabei kann vorgesehen sein, dass jedem Fixierelement ein Aufnahmeelement zugeordnet ist. Es ist aber auch denkbar, dass jedem Fixierelement eine Mehrzahl von Aufnahmeelementen zugeordnet sein kann, wobei die Aufnahmeelemente im Wesentlichen in linearer Anordnung vom Labialbereich zum Rachenbereich hin verlaufend auf der jeweiligen Schiene angeordnet sind. Die Vorrichtung kann somit verschiedenen Personen beziehungsweise verschiedenen Kieferstellungen angepasst werden.

Eine bevorzugte Ausführungsform der Erfindung sieht vor, dass die die Aufnahmeelemente aufweisende Schiene bereichsweise aus der Bissfläehe hervorragende Erhöhungen aufweist, in deren zur jeweils gegenüberliegenden Schiene weisenden Fläche die Aufnahmeelemente eingeformt sind. Dies hat den Vorteil, dass während der Behandlung beziehungsweise während des Anpassvorgangs, durch die zwischen den Erhöhungen entstehenden Bereiche eine uneingeschränkte Atmungsfunktion beziehungsweise Luftzirkulation für die die Vorrichtung tragende Person gewährleistet werden kann.

Eine Ausführungsform sieht dabei vor, dass die thermoplastische Formmasse im Wesentlichen aus einem Ethylen-Vinylacetat-Copolymer besteht, wobei sich dieses Material durch eine gute Verträglichkeit und hervorragende Verarbeitbarkeit auszeichnet.

Nachfolgend wird eine bevorzugte Ausführungsform der Vorrichtung an Hand der Zeichnungen näher erläutert.

Es zeigen:
- **Fig. 1**: eine erfindungsgemäße Vorrichtung umfassend eine Ober- und eine Unterkieferschiene in perspektivischer Darstellung;
- **Fig. 2**: die Oberkieferschiene gemäß **Fig. 1** in einer Einzeldarstellung;
- **Fig. 3**: die Oberkieferschiene in einer Ansicht von unten;
- **Fig. 4**: die Unterkieferschiene gemäß **Fig. 1** in einer Einzelansicht;
- **Fig. 5**: die Unterkieferschiene in einer Ansicht von unten.

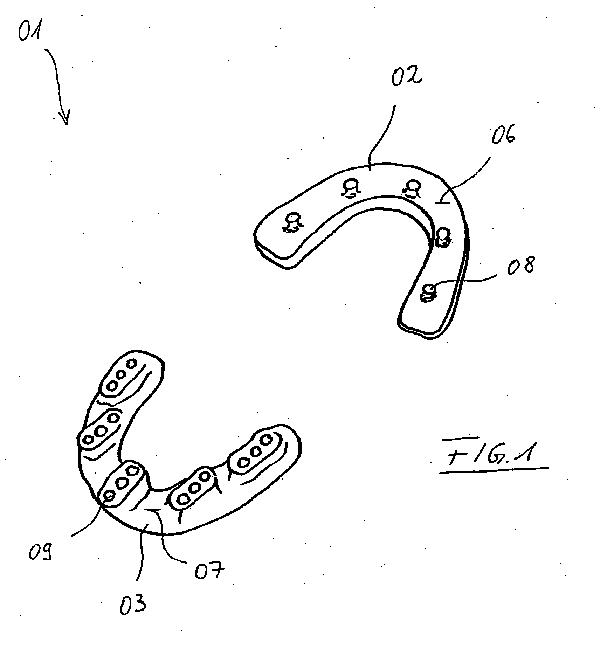

**Fig. 1** zeigt eine Vorrichtung 01 aufweisend eine Oberkieferschiene 02 und eine Unterkieferschiene 03. Beide Schienen sind im wesentlichen U-förmig ausgebildet und können jeweils auf den oberen beziehungsweise unteren Zahnbogen eines Benutzers zumindest bereichsweise aufgesetzt werden.

Sowohl die Oberkieferschiene 02 als auch die Unterkieferschiene 03 wiesen hier, nicht näher dargestellten beziehungsweise nicht erkennbaren Aufnahmebereichen 04, 05 gegenüberliegenden, Bissflächenbereiche 06, 07 auf, wobei auf den Bissflächenbereichen 06 und 07 eine Fixiereinrichtung vorgesehen ist. Auf der Oberkieferschiene 02 beziehungsweise auf dem Bissflächenbereich 06 sind eine Mehrzahl von Fixierelementen 08 vorgesehen, wobei die Fixierelemente 08 hierbei im wesentlichen einstückig mit der Oberkieferschiene 02 beziehungsweise dem Bissflächenbereich 06 hergestellt sind.

Auf der Unterkieferschiene 03 beziehungsweise auf dem Bissflächenbereich 07 der Unterkieferschiene 03 sind Aufnahmeelemente 09 vorgesehen, wobei auch die Aufnahmeelemente 09 einstückig mit der Unterkieferschiene 03 beziehungsweise mit dem Bissflächenbereich 07 hergestellt sind. Die Darstellung der hier abgebildeten Vorrichtung 01 beziehungsweise der an der Vorrichtung 01 vorgesehenen Fixiereinrichtung, gebildet vom Fixierelement 08 und dem Aufnahmeelement 09, erfolgt beispielhaft, dergestalt, dass das Fixierelement 08 an der Oberkieferschiene 02 und das Aufnahmeelement 09 an der Unterkieferschiene 03 vorgesehen ist. Es ist aber auch denkbar, die Fixiereinrichtung umgekehrt, so dass das Fixierelement 08 an der Unterkieferschiene 03 und das Aufnahmeelement 09 an der Oberkieferschiene 02 vorgesehen ist, auszubilden. Erfindungsgemäß macht dies jedoch keinen Unterschied, da bei beiden Varianten ein sicherer Sitz beziehungsweise eine zuverlässige Positionierung der beiden Schienen zueinander gewährleistet werden kann.

Die hier abgebildete Vorrichtung 01 kann sowohl zur Unterstützung und Therapieplanung, als Testgerät zur Feststellung der Akzeptanz und Verträglichkeit einer intraoralen Vorrichtung oder auch als kurzfristig verfügbares Ersatzgerät bei Ausfall eines vorhandenen intraoralen Therapiegeräts fungieren. Erfindungsgemäß ist die Vorrichtung 01 deshalb aus einer verformbaren, bei Raumtemperatur festen, mäßig harten, bei Erwärmung plastifizierbaren, eine niedrige Erweichungstemperatur aufweisenden thermoplastischen Formmasse hergestellt. Es ist somit möglich, die Vorrichtung 01 kurzfristig so an den Benutzer anzupassen, dass eine aufwendige Anpassung beziehungsweise das umständliche Abnehmen einer beispielsweise gipsartigen Positivform, wie aus dem Stand der Technik bekannt, entfallen kann. Die Vorrichtung 01 kann somit als einfaches und günstig herzustellendes Testgerät fungieren, mit der der gewünschte Effekt der Vorrichtung beim Benutzer getestet werden kann. Die Vorrichtung 01 kann also im positiven Sinne beispielsweise in der Art eines kostengünstig herstellbaren, einfach anzulegenden einmal zu benutzenden "Wegwerfartikel" dienen, mit dessen Hilfe die Wirksamkeit einer Dauertherapie mit einem kostspieligerem intraoralem Gerät prognostizierbar wird.

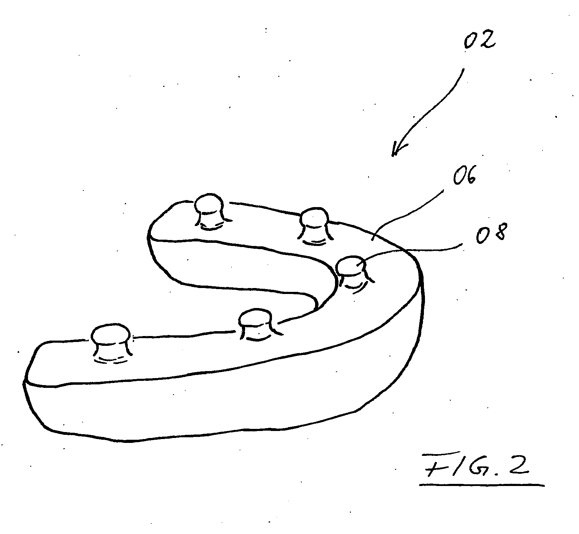

**Fig. 2** zeigt die Oberkieferschiene 02 gemäß Vorrichtung 01 in einer Detail- beziehungsweise Einzelansicht. Die Oberkieferschiene 02 mit den auf dem Bissflächenbereich 06 vorgesehenen Fixierelementen 08 ist einstückig hergestellt und weist einen dem Bissflächenbereich gegenüberliegenden, nicht näher dargestellten, Aufnahmebereich für den Zahnbogen eines Benutzers auf.

Die Fixierelemente 08 selber sind im wesentlichen pilzartig ausgebildet und können nach Art einer lösbaren Rastverbindung in das zugeordnete Aufnahmeelement in der Unterkieferschiene eingesteckt werden. Bei der hier abgebildeten Oberkieferschiene 02 sind fünf Fixierelemente 08 auf dem Bissflächenbereich 06 vorgesehen. Jedem Fixierelement 08 ist zumindest ein Aufnahmeelement auf der Unterkieferschiene zugeordnet.

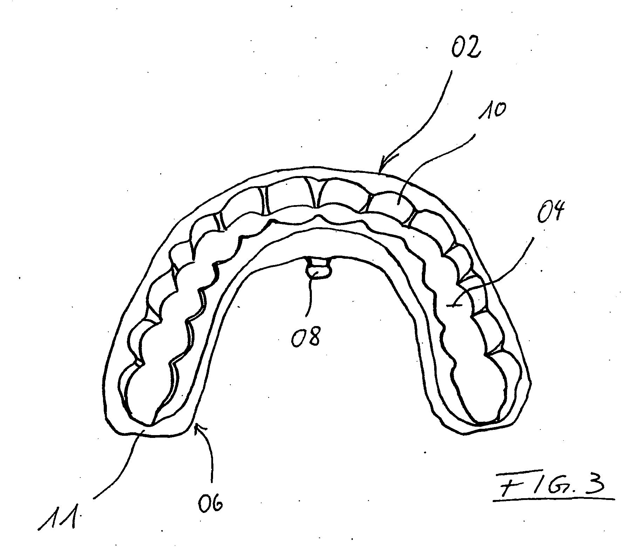

Durch die pilzartige Ausgestaltung der Fixierelemente 08 kann eine sehr sichere im wesentlichen formschlüssige Steckverbindung, mit sehr guten Halte- beziehungsweise Fixiereigenschaften, auch bei größeren Verschiebekräften der beiden Schienen zueinander, gewährleistet werden. Fig. 3 zeigt die Oberlcieferschiene 02 in einer Ansicht von unten. Deutlich zu erkennen ist der Aufnahmebereich 04, in den der Zahnbogen eines Benutzers diesen zumindest bereichsweise umgebend, aufsetzbar ist. Der Aufnahmebereich 04 weist vorgefertigte Abformungen 10 verschiedener Zahnformen auf und kann beim Anlegen, durch leichtes Drücken, der Zahnform beziehungsweise dem Zahnbogen des Benutzers angepasst werden. Der Aufnahmebereich 04 ist bevorzugt so ausgelegt das er für eine Vielzahl von unterschiedlichen Zahnbögen beziehungsweise Zahnformen eingesetzt werden kann. Es ist aber auch denkbar auf das Vorsehen eines Aufnahmebereichs 04 zu verzichten und die Oberkieferschiene 02 vollmassig auf den Zahnbogen eines Benutzers zu pressen.

Bei Vorsehen eines Aufnahmebereichs 04 ist eine ausreichende Materialstärke 11 zwischen Aufnahmebereich 04 und Bissflächenbereich 06 vorgesehen, um einen sicheren Sitz des Fixierelements 08 zu gewährleisten.

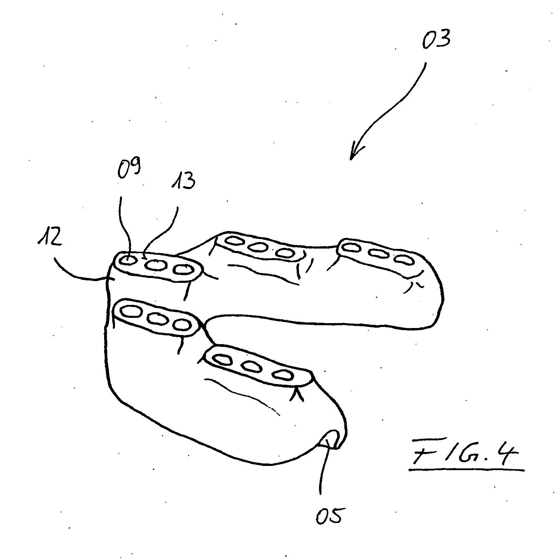

**Fig. 4** zeigt die Unterkieferschiene 03 gemäß Vorrichtung 01 in einer Detail- beziehungsweise Einzelansicht. Die Unterkieferschiene 03 ist auf ihrem Bissflächenbereich 07 mit Aufnahmeelementen 09 versehen, wobei jedem Fixierelement eine Mehrzahl von Aufnahmeelementen 09 zugeordnet ist. Die Aufnahmeelemente 09 sind im wesentlichen in linearer Anordnung vom Labialbereich zum Rachenraum hin verlaufend auf der Unterkieferschiene 03 angeordnet.

Darüber hinaus weist die Unterkieferschiene 03 bereichsweise aus dem Bissflächenbereich 07 hervorragende Erhöhungen 12 auf, in deren zur gegenüberliegenden Schiene weisenden Fläche 13 die Aufnahmeelemente 09 eingeformt sind.

Bei der hier abgebildeten Ausführungsform der Unterkieferschiene 03 sind in jeder Erhöhung 12 drei Aufnahmeelemente 09 vorgesehen. Jedem der Fixierelement 08 auf der Oberkieferschiene 02 ist also eine Mehrzahl, hier also drei, von Aufnahmeelementen 09 zugeordnet. Es kann also eine Positionsanpassung beim Anlegen der Vorrichtung im Mund eines Benutzers in Bezug auf den Sitz der Oberkieferschiene zur Unterkieferschiene, oder umgekehrt, realisiert werden.

Das Aufnahmeelement 09 ist bevorzugt so ausgebildet, dass es im Wesentlichen der Form beziehungsweise Ausgestaltung des in das Aufnahmeelement 09 einsteckbaren Fixierelements 08 entspricht. Dadurch wird eine im Wesentlichen formschlüssige Verbindung gebildet.

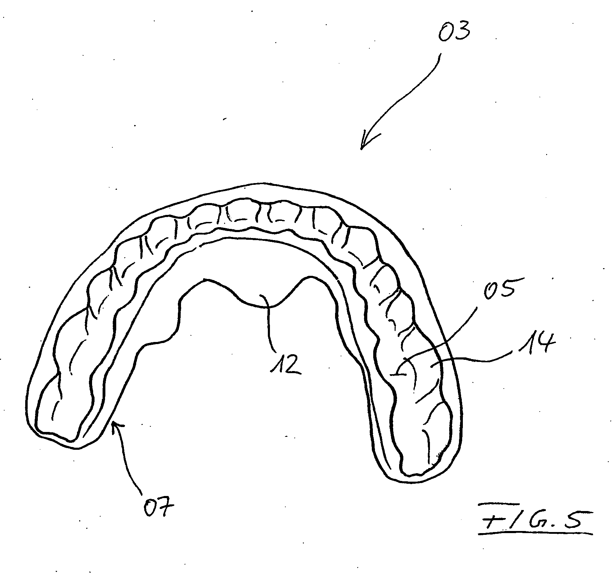

Fig. 5 zeigt die Unterkieferschiene 03 in einer Ansicht von unten. Deutlich zu erkennen ist der Aufnahmebereich 05 der vorgefertigte Abformungen 14 verschiedener Zahnformen aufweist. Ebenfalls deutlich zu erkennen sind die auf dem Bissflächenbereich 07 liegenden Erhöhungen 12 in denen die Aufnahmeelemente 09 eingeformt sind.

## Patentansprüche

1. Intraorale Vorrichtung (1), insbesondere zum Einsatz bei schlafbezogenen Atmungsstörungen, mit einer im wesentlichen U-förmig ausgebildeten Oberkieferschiene (02), die auf den oberen Zahnbogen eines Benutzers, diesen zumindest bereichsweise umgebend, aufsetzbar ist, und einer ebenfalls im wesentlichen U-förmig ausgebildeten Unterkieferschiene (03), die auf den unteren Zahnbogen eines Benutzers, diesen zumindest bereichsweise umgebend, aufsetzbar ist, wobei die Ober- (02) und die Unterkieferschiene (03) jeweils zueinander weisende Bissflächenbereiche (06, 07) aufweisen, in deren Bereich zumindest eine Fixiereinrichtung vorgesehen ist, mit der die Unterkieferschiene (03) in einer vorbestimmten relativen Position zur Oberkieferschiene (02) fixierbar ist, wobei die Fixiereinrichtung zumindest ein Fixierelement (08) und zumindest ein das Fixierelement (08) zumindest bereichsweise aufnehmendes Aufnahmeelemente (09) aufweist, wobei das Fixierelement (08) an der Oberkieferschiene (02) und das Aufnahmeelement (09) an der Unterkieferschiene (03) oder, umgekehrt, das Fixierelement (08) an der Unterkieferschiene (03) und das Aufnahmeelement (09) an der Oberkieferschiene (02) vorgesehen ist, wobei Fixierelement (08) und Aufnahmeelement (09) eine im wesentlichen formschlüssige Steckverbindung bilden,
**dadurch gekennzeichnet,**
**dass** Ober- und/oder Unterkieferschiene (02, 03) einschließlich der Fixier (08)- und/oder Aufnahmeelemente (09) jeweils einstückig aus einer verformbaren, bei Raumtemperatur festen, mäßig harten, bei Erwärmung plastifizierbaren, eine niedrige Erweichungstemperatur aufweisenden thermoplastischen Formmasse hergestellt sind.

2. Intraorale Vorrichtung nach Anspruch 1,
**dadurch gekennzeichnet,**
**dass** das Fixierelement (08) im wesentlichen pilz- und/oder zapfenartig ausgebildet ist und nach Art einer lösbaren Rastverbindung in das zugeordnete Aufnahmeelement (09) einsteckbar ist.

3. Intraorale Vorrichtung nach Anspruch 1 oder 2,
**dadurch gekennzeichnet,**
**dass** die Steckverbindung im wesentlichen als druckknopfartige Verbindung ausgebildet ist.

4. Intraorale Vorrichtung nach einem der Ansprüche 1 bis 3,
**dadurch gekennzeichnet,**
**dass** mindestens zwei, insbesondere zwei bis zehn Fixierelemente (08) vorgesehen sind.

5. Intraorale Vorrichtung nach einem der Ansprüche 1 bis 4,
**dadurch gekennzeichnet,**
**dass** jedem Fixierelement (08) ein Aufnahmeelement (09) zugeordnet ist.

6. Intraorale Vorrichtung nach einem der Ansprüche 1 bis 4,
**dadurch gekennzeichnet,**
**dass** jedem Fixierelement (08) eine Mehrzahl von Aufnahmeelementen (09) zugeordnet ist, wobei die Aufnahmeelemente (09) im wesentlichen in linearer Anordnung vom Labialbereich zum Rachenbereich hin verlaufend auf der jeweiligen Schiene angeordnet sind.

7. Intraorale Vorrichtung nach einem der Ansprüche 1 bis 6,
**dadurch gekennzeichnet,**
**dass** die die Aufnahmeelemente (09) aufweisende Schiene bereichsweise aus der Bissfläche (06, 07) hervorragende Erhöhungen (12) aufweist, in deren zur jeweils gegenüberliegenden Schiene weisenden Fläche (13) die Aufnahmeelemente (09) eingeformt sind.

8. Intraorale Vorrichtung nach einem der Ansprüche 1 bis 7,
**dadurch gekennzeichnet,**
**dass** die thermoplastische Formmasse im wesentlichen aus einem Ethylen-Vinylacetat-Copolymer besteht.

## Claims

1. An intraoral device (01), in particular for use with sleep-related breathing disorders, having a maxillary splint (02) designed to be essentially U-shaped, which can be placed on the user's alveolar arch of maxilla, surrounding it in at least some areas, and a mandibular splint (03) which is also designed to be essentially U-shaped and can be placed on the user's alveolar arch of mandible, surrounding it in at least some areas, wherein the maxillary splint (02) and the mandibular splint (03) have bite surface areas (06, 07) facing one another, at least one fixation device being provided in their area, with which the mandibular splint (03) can be secured in a predetermined position relative to the maxillary splint (02), wherein the fixation device comprises at least one fixation element (08) and at least one receptacle element (09) which accommodates the fixation element (08) in at least some areas, wherein the fixation element (08) is provided on the maxillary splint (02), and the receptacle element (09) is provided on the mandibular splint (03), or conversely, the fixation element (08) is provided on the mandibular splint (03) and the receptacle element (09) is provided on the maxillary splint (02), wherein the fixation element (08) and the receptacle element (09) form an essentially form-fitting plug-type connection,
**characterised in that**
the maxillary splint and/or mandibular splint (02, 03) including the fixation elements (08) and/or receptacle elements (09) are each manufactured in one piece from a thermoplastic moulding compound that is formable, is solid at room temperature, is moderately hard, can be plastified on heating and has a low softening temperature.

2. The intraoral device according to Claim 1,
**characterised in that**
the fixation element (08) is designed to be essentially conical and/or mushroom-shaped and can be inserted into the respective receptacle element (09) in the manner of a releasable catch connection.

3. The intraoral device according to Claim 1 or 2,
**characterised in that**
the plug-type connection is designed to be essentially a pushbutton-type connection.

4. The intraoral device according to any one of Claim 1 through 3,
**characterised in that**
at least two, in particular two to ten fixation elements (08) are provided.

5. The intraoral device according to any one of Claims 1 through 4,
**characterised in that**
a receptacle element (09) is assigned to each fixation element (08).

6. The intraoral device according to any one of Claims 1 through 4,
**characterised in that**
a plurality of receptacle elements (09) is assigned to each fixation element (08), wherein the receptacle elements (09) are arranged on the respective splint essentially in a linear arrangement running from the labial area to the pharyngeal area.

7. The intraoral device according to any one of Claims 1 through 6,
**characterised in that**
the splint having the receptacle elements (09) has elevations (12) protruding out of the bite surface (06, 07) in some areas, the receptacle elements (09) being moulded in their surface (13) opposite the respective splint.

8. The intraoral device according to any one of Claims 1 through 7,
**characterised in that**
the thermoplastic moulding compound is comprised essentially of an ethylene-vinyl acetate copolymer.

## Revendications

1. Dispositif intrabuccal (01), en particulier à utiliser en cas de troubles respiratoires liés au sommeil, ayant une attelle maxillaire (02) conçue pour être essentiellement en forme de U et qui peut être placée sur l'arcade dentaire supérieure d'un utilisateur, entourant celle-ci dans au moins certaines zones, et une attelle mandibulaire (03) qui est aussi essentiellement en forme de U et peut être placée sur l'arcade dentaire inférieure de l'utilisateur, entourant celle-ci dans au moins certaines zones, l'attelle maxillaire (02) et l'attelle mandibulaire (03) ayant des zones de surfaces occlusales (06, 07) qui sont disposées en vis-à-vis et dans lesquelles au moins un dispositif de fixation est prévu avec lequel l'attelle mandibulaire (03) peut être fixée dans une position prédéterminée relative à l'attelle maxillaire (02), le dispositif de fixation comprenant au moins un élément de fixation (08) et au moins un élément de réception (09) qui accueille l'élément de fixation (08) dans au moins certaines zones, l'élément de fixation (08) étant prévu sur l'attelle maxillaire (02) et l'élément de réception (09) étant prévu sur l'attelle mandibulaire (03) ou, à l'envers, l'élément de fixation (08) étant prévu sur l'attelle mandibulaire (03) et l'élément de réception (09) étant prévu sur l'attelle maxillaire (02), l'élément de fixation (08) et l'élément de réception (09) formant une connexion enfichable essentiellement par engagement positif,
**caractérisé en ce que**
l'attelle maxillaire (02) et/ou l'attelle mandibulaire (03), y compris les éléments de fixation (08) et/ou les éléments de réception (09), sont produites chacune d'une seule pièce, d'une matière à mouler thermoplastique qui est moulable, qui est solide à température ambiante, qui est modérément dure, qui peut être plastifiée lors du chauffage et qui a une température de ramollissement basse.

2. Dispositif intrabuccal selon la revendication 1,
**caractérisé en ce que**
l'élément de fixation (08) est conçu pour être essentiellement en forme de champignon et/ou de pin et peut être enfiché dans l'élément de réception (09) correspondant à la mode d'une connexion par encliquetage amovible.

3. Dispositif intrabuccal selon l'une des revendications 1 ou 2,
**caractérisé en ce que**
la connexion enfichable est conçue pour être essentiellement une connexion de type bouton-poussoir.

4. Dispositif intrabuccal selon l'une des revendications 1 à 3,
**caractérisé en ce qu'**
au moins deux, en particulier deux à dix éléments de fixation (08) sont prévus.

5. Dispositif intrabuccal selon l'une des revendications 1 à 4,
**caractérisé en ce qu'**
un élément de réception (09) est assigné à chaque élément de fixation (08).

6. Dispositif intrabuccal selon l'une des revendications 1 à 4,
**caractérisé en ce qu'**
une multitude d'éléments de réception (09) est assignée à chaque élément de fixation (08), les éléments de réception (09) étant disposés sur l'attelle correspondante essentiellement dans une disposition linéaire qui passe de la zone labiale à la zone pharyngée.

7. Dispositif intrabuccal selon l'une des revendications 1 à 6,
**caractérisé en ce que**
l'attelle ayant l'élément de réception (09) comprend des élévations (12) qui dépassent de la surface occlusale (06, 07) dans certaines zones, les éléments de réception (09) étant moulés chacun dans la surface (13) desdites élévations en face de l'attelle correspondante.

8. Dispositif intrabuccal selon l'une des revendications 1 à 7,
**caractérisé en ce que**
la matière à mouler thermoplastique se compose essentiellement d'un copolymère éthylène-acétate de vinyle.
